# EUROPÄISCHE PATENTANMELDUNG

(11) **EP 4 847 623 A1**
(43) Veröffentlichungstag der Anmeldung: **02.12.2026**
(21) Anmeldenummer: 25179837.7
(22) Anmeldetag: 30.05.2025
(51) Int. Cl.: C12P 7/6409, C12P 7/6431, C12P 7/6463, C12P 7/6472, C12N 1/16, A61K 8/36, A61Q 19/00, C12R 1/73

(54) **FERMENTATIONSVERFAHREN ZUR ERZEUGUNG EINER FETTSÄURENZUSAMMENSETZUNG**

(71) Anmelder: Mibelle AG, 5033 Buchs (CH); Fraunhofer-Gesellschaft zur Förderung der angewandten Forschung e.V., 80686 München (DE)
(72) Erfinder: Heldmaier, Susanne, 5033 Buchs (CH); Mastrobuoni, Luciano, 5033 Buchs (CH); Söllner, Bernd, 5033 Buchs (CH); Wegat, Vanessa, 94315 Straubing (DE); Speck, Melanie, 94315 Straubing (DE); Fabarius, Jonathan Thomas, 94315 Straubing (DE); Roth, Arne, 94315 Straubing (DE)
(74) Vertreter: Hepp Wenger Ryffel AG

(57) **Zusammenfassung**

Die Erfindung betrifft ein Fermentationsverfahren zur Erzeugung einer Fettsäurenzusammensetzung, umfassend die Schritte a) Bereitstellen eines Fermentationsmediums, das Ethanol und Kaliumionen enthält; und b) Durchführung einer Fermentation mittels einer Ölhefe in dem Fermentationsmedium. Weiter betrifft die Erfindung die Verwendung von Kaliumionen zur Beeinflussung der Massenanteile von Fettsäuren in dem erfindungsgemässen Fermentationsverfahren. Zudem betrifft die Erfindung kosmetische Zusammensetzungen enthaltend die erfindungsgemässe Fettsäurenzusammensetzung.

## Beschreibung

Die Erfindung betrifft ein Fermentationsverfahren zur Erzeugung einer Fettsäurenzusammensetzung umfassend das Bereitstellen eines Fermentationsmediums, das Ethanol und Kaliumionen enthält und die Durchführung einer Fermentation mittels einer Ölhefe in dem Fermentationsmedium. Weiter betrifft die Erfindung die Verwendung von Kaliumionen zur Beeinflussung der Massenanteile von Fettsäuren in dem erfindungsgemässen Fermentationsverfahren. Zudem betrifft die Erfindung kosmetische Zusammensetzungen enthaltend die oben genannten Fettsäurezusammensetzungen.

Im Stand der Technik ist bekannt, dass Ölhefen, wie *Yarrowia Lipolytica*, in einer Vielzahl von biotechnologischen Prozessen einsetzbar sind. Dazu gehört gemäss Goncalves et al. (G. GONCALVES et al., Yarrowia lipolytica and Its Multiple Applications in the Biotechnological Industry. Sci. World J., March 2014) die Produktion von Biotensiden, Biotreibstoffen, Citrat, Lactonen, Lipiden, Fettsäuren und Lipasen. Übliche Kohlenstoffquellen für die Produktion von Fettsäuren durch Ölhefen im Stand der Technik sind gemäss Nambou et al. (NAMBOU et al., Designing of a "cheap to run" fermentation platform for an enhanced production of single cell oil from Yarrowia lipolytica DSM3286 as a potential feedstock for biodiesel., Bioresour. Technol., December 2014, Vol. 173, pages 324 - 333) Glukose, Saccharose, Fruktose, Xylose, Maltose, Trehalose, L-Malat. Ein Problem dieser Kohlenstoffquellen ist, dass sie mit der Nutzung von Ackerflächen verbunden sind. Das führt zu einer Abhängigkeit von natürlichen Bedingungen sowie begrenzter Skalierbarkeit und Flexibilität bei der industriellen fermentativen Herstellung von Fettsäuren durch Ölhefen. Oft werden die genannten Kohlenstoffquellen aus Palmöl oder Zuckerrohr gewonnen. Diese werden typischerweise in tropischen Regionen angepflanzt. Für die grossflächige Kultivierung wird der Regenwald gerodet, was negative Auswirkungen auf die Biodiversität und das Klima hat. Ein weiteres Problem ist, dass die gewählte Kohlenstoffquelle zu einer geringen Ausbeute führen kann. Mögliche Gründe sind hierbei die Entstehung von Nebenprodukten oder auch eine geringe Bioverfügbarkeit der Kohlenstoffquelle.

Goncalves et al. haben Methanol als Kohlenstoffquelle für die Herstellung von Lipiden beschrieben, Ethanol hingegen wird in diesem Kontext sehr selten genannt. Der Bildung von NAD(P)H aus NAD(P)⁺ während der Fermentation durch die Ölhefe mit Methanol als Substrat wird eine Gibbs Reaktionsenergie von - 972.5 kJ/mol zugeordnet. Bei Ethanol beträgt dieser Wert lediglich - 905.8 kJ/mol. Die Reaktion mit Methanol ist somit gegenüber der Reaktion mit Ethanol energetisch begünstigt. Ethanol wird in DE10333144B4 als Kohlenstoffquelle zur fermentativen Herstellung von Citrat durch *Yarrowia Lipolytica* beschrieben. Die Nutzung von Ethanol als Kohlenstoffquelle für die Herstellung von Fettsäuren durch Ölhefen wurde im Stand der Technik bisher nicht systematisch untersucht.

Fettsäuren sind in einer Vielzahl von Industrien einsetzbar, wie beispielsweise in der Nahrungsmittel- oder Kosmetikindustrie. Man unterscheidet zwischen gesättigten und ungesättigten Fettsäuren. Hierbei werden ungesättigte Fettsäuren bevorzugt, da diesen besonders vorteilhafte Eigenschaften zugeschrieben werden. Weiter werden die ungesättigten Fettsäuren in einfach und mehrfach ungesättigte Fettsäuren unterteilt. Die Produkte fermentativer Fettsäuren-Herstellungsverfahren durch Ölhefen beinhalten stets eine Mischung aus unterschiedlichen Fettsäuren, also eine Fettsäurenzusammensetzung mit Fettsäuren welche unterschiedlich lange Kohlenstoffketten und Sättigungsgrade haben. Da die Fettsäuren in den Zusammensetzungen meist sehr ähnliche chemische und physikalische Eigenschaften aufweisen, ist es kaum möglich diese Zusammensetzungen auf eine ökonomisch sinnvolle Weise in die einzelnen Fettsäuren aufzutrennen, wie beispielsweise durch Nimesh et al. (NIMESH P. R. et al., Separation of C18 Fatty Acid Esters and Fatty Acids Derived from Vegetable Oils Using Nanometer-Sized Covalent Organic Frameworks Incorporated in Polyepoxy Membranes, ACS Appl. Nano Mater., April 2023, Vol. 6, No. 8, pages 6715 - 6725.) beschrieben wird. Obwohl für gewisse Anwendungen isolierte Fettsäuren vorteilhaft wären, wird im Stand der Technik meist die gesamte Fettsäurenzusammensetzung entlang der weiteren Wertschöpfungskette genutzt, da sich eine Auftrennung ökonomisch nicht lohnt. Eine Auftrennung ist auch aus ökologischer Sicht bedenklich, da hier oft isolierte Bestandteile einer Zusammensetzung verwertet werden, anstatt alle Bestandteile zu nutzen. Es ist daher wichtig, dass die Zusammensetzung einen möglichst hohen Massenanteil an hochwertigen Fettsäuren enthält. Der Massenanteil bezeichnet (auch im Rahmen der vorliegenden Erfindung) den prozentualen Anteil einer bestimmten Fettsäure oder einer Fettsäureart an der Gesamtmasse aller in der Zusammensetzung gemessenen Fettsäuren. (Die experimentelle Bestimmung des Massenanteils wird nachstehend in Ausführungsbeispiel 1 beschreiben.)

Es existieren im Stand der Technik kaum Ansätze, die Fettsäurezusammensetzung während des Fermentationsverfahrens durch die Ölhefe systematisch zu beeinflussen, sodass man einen höheren Massenanteil einer bevorzugten Fettsäure erhält. Das führt dazu, dass im Stand der Technik oft die Gesamtausbeute der Fettsäurenzusammensetzung im Zentrum steht, während die Qualität der Fettsäuren nur hintergründig beachtet wird. Aus diesem Grund finden sich im Stand der Technik oft Fettsäurezusammensetzungen, welche qualitativ nicht optimiert sind. Je nach Anwendung kann das bedeuten, dass sie einen hohen Massenanteil an gesättigten Fettsäuren enthalten, oder dass innerhalb der ungesättigten Fettsäuren qualitativ hochwertigere Fettsäuren, wie zum Beispiel die mehrfach ungesättigte Linolsäure (CAS Nr. 60-33-3), wenig vertreten sind. Ölsäure (CAS Nr. 112-80-1) ist ein Beispiel für eine (nur) einfach ungesättigte Fettsäure. Der menschliche Körper ist in der Lage, einfach ungesättigte Fettsäuren selbst zu synthetisieren, nicht jedoch mehrfach ungesättigte Fettsäuren. Es ist somit erstrebenswert, eine Fettsäurezusammensetzung herzustellen, welche einen hohen Massenanteil an mehrfach ungesättigten Fettsäuren aufweist. Im Stand der Technik sind die Massenanteile der mehrfach ungesättigten Fettsäuren jedoch meist unter 15 Gew. %.

Es ist die Aufgabe der vorliegenden Erfindung, zumindest einige, wenn nicht alle vorgenannten Nachteile des Stands der Technik zu überwinden.

Die Aufgabe wird durch das erfindungsgemässe Fermentationsverfahren gemäss den unabhängigen Ansprüchen gelöst. Vorteilhafte Ausgestaltungen und Weiterbildungen sind Gegenstand der abhängigen Ansprüche.

Ein erster Aspekt der Erfindung betrifft ein Fermentationsverfahren zur Erzeugung einer Fettsäurezusammensetzung umfassend die Schritte: a) Bereitstellen eines Fermentationsmediums, das Ethanol und Kaliumionen enthält und b) die Durchführung einer Fermentation mittels einer Ölhefe in dem Fermentationsmedium.

Überraschenderweise wurde festgestellt, dass die Nutzung von Ethanol (CAS Nr. 64-17-5) als Kohlenstoffquelle in Kombination mit Kaliumionen für die fermentative Herstellung einer Fettsäurenzusammensetzung durch eine Ölhefe in einer hochwertigen Fettsäurenzusammensetzung in zufriedenstellender Ausbeute resultiert. Diese hat einen hohen Massenanteil an ungesättigten Fettsäuren (unter anderem Ölsäure und Linolsäure), wobei insbesondere der Massenanteil der mehrfach ungesättigten Fettsäuren, insbesondere der Linolsäure, überraschend hoch ist. Durch das erfindungsgemässe Fermentationsverfahren konnte ein Massenanteil der Linolsäure von über 34 Gew. % der Fettsäurenszusammensetzung erreicht werden. Die Nutzung von Ethanol und Kaliumionen resultiert also in einem überraschend hohen Massenanteil der Linolsäure.

Die durch das Fermentationsverfahren erhaltene Fettsäurenzusammensetzung umfasst insbesondere die folgenden Fettsäuren: Palmitinsäure (CAS Nr. 57-10-3), Palmitoleinsäure (CAS Nr. 373-49-9), Ölsäure, Stearinsäure (CAS Nr. 57-11-4) und Linolsäure. Dabei machen die Ölsäure und die Linolsäure den grössten Anteil der Gesamtmassen der Fettsäuren aus. Es finden sich auch andere Fettsäuren.

Das Fermentationsmedium kann weitere Komponenten enthalten einschliesslich, aber nicht beschränkt auf: KCl (CAS Nr. 7447-40-7), Ethanol, NaH₂PO₄ (CAS Nr. 7558-80-7), Na₂HPO₄ x 2 H₂O (CAS Nr. 7558-79-4), MgSO₄ (CAS Nr. 7487-88-9), (NH₄)₂SO₄ (CAS Nr. 7783-20-2), CaCl₂ (CAS Nr. 10043-52-4), FeCl₃ (CAS Nr. 7705-08-0), ZnSO₄ (CAS Nr. 7733-02-0), MnSO₄ (CAS Nr. 7785-87-7), CoCl₂ (CAS Nr. 7646-79-9), Thiamin-HCL (CAS Nr. 67-03-8).

Die erhaltenen Fettsäuren liegen nach dem Fermentationsverfahren in der Regel in der Form von Triglyceriden und freien Carbonsäuren vor.

In einer bevorzugten Ausführungsform des erfindungsgemässen Fermentationsverfahrens beträgt die Konzentration der Kaliumionen im Fermentationsmedium 0.5 mM - 150 mM, bevorzugt 30 mM - 110 mM, besonders bevorzugt 50 mM - 80 mM.

Es wurde festgestellt, dass über die Konzentration der Kaliumionen im Fermentationsmedium die Massenanteile der Fettsäuren in der Fettsäurenzusammensetzung beeinflusst werden kann. Im Konzentrationsbereich von 0.5 mM - 150 mM ist eine höhere Kaliumionenkonzentration allgemein mit einer höheren Fettsäurenausbeute verbunden. Es wurde überraschend festgestellt, dass bei niedrigeren Kaliumionenkonzentrationen ein höherer Massenanteil der mehrfach ungesättigten Fettsäuren, insbesondere der Linolsäure, erreicht wird. Es wurden Steigerungen des Linolsäuregehalts auf über 34 Gew. % beobachtet. Ein erhöhter Massenanteil der Linolsäure ist von Vorteil, da die Linolsäure eine zentrale Rolle bei der Synthese von Ceramid I spielt, einem strukturell wichtigen Bestandteil der epidermalen Barrierefunktion. Zudem wird ein Rückgang des Linolsäuregehalts in der Haut mit dem Auftreten von atopischer Dermatitis in Verbindung gebracht. Die topische Applikation von Linolsäure kann zur Regeneration der Hautbarriere beitragen. Weiter kann auch ein erhöhter Massenanteil der Ölsäure vorteilhaft sein, welche dafür bekannt ist, die Penetration der Hautbarriere zu fördern, was die Haut aufnahmefähiger macht. Je nach Anwendung der erfindungsgemässen Fettsäurezusammensetzung kann somit entweder die Gesamtmenge oder die Qualität der Fettäurenzusammensetzung über die Steuerung der Kaliumionenkonzentration erhöht werden. Im Bereich zwischen 50 mM und 80 mM konnte eine besonders zufriedenstellende Fettsäurenzusammensetzung erreicht werden.

In einer bevorzugten Ausführungsform des erfindungsgemässen Fermentationsverfahrens umfasst Schritt a) das Auflösen eines Kaliumsalzes, wobei das Kaliumsalz ausgewählt ist aus der Gruppe bestehend aus KCl, K₂CO₃, KOH, K₂SO₄, K₃PO₄, CH₃COOK, C₆H₅K₃O₇ und HCOOK.

Die Art des Kaliumsalzes kann einen Einfluss auf das Fermentationsverfahren haben. Die Art des Kaliumsalzes ist je nach Anwendung wählbar. Es wurde gefunden, dass es für den Abbau des Ethanols von Vorteil ist, wenn der Ölhefe, insbesondere *Yarrowia Lipolytica,* im Fermentationsmedium keine zusätzliche Kohlenstoffquelle zur Verfügung steht. KCl ist ein bevorzugtes Kaliumsalz, da es keine Kohlenstoffquelle darstellt und es sich sehr gut im Fermentationsmedium lösen lässt. Weiter ist KCl als Kaliumsalz mit wenig Nebeneffekten verbunden. K₂SO₄ (CAS Nr. 7778-80-5) kann vorteilhafterweise eine Sulfatquelle für die Ölhefe darstellen. Weiter weist K₂SO₄ gegenüber KCl den Vorteil auf, dass das während des Fermentationsverfahrens entstehende Abwasser mit weniger Aufwand entsorgt werden kann. K₃PO₄ (CAS Nr. 7778-53-2) hat den Vorteil, dass es neben dem Kalium auch Phosphat als Nährstoff liefern kann. Zudem kann K₃PO₄ puffernde Eigenschaften aufweisen, was von Vorteil für das Fermentationsverfahren sein kann.

In einer bevorzugten Ausführungsform wird das erfindungsgemässe Fermentationsverfahrens als Chargenprozess (auch als Batchproduktion bekannt) durchgeführt. Dabei kann die Anfangskonzentration des Ethanols im Fermentationsmedium insbesondere 2.5 g/L - 100.0 g/L oder 2.5 g/L - 50.0 g/L oder 2.5 g/L - 15.0 g/L, bevorzugt 5.0 g/L - 13.0 g/L, besonders bevorzugt 8.0 g/L - 11.0 g/L betragen.

In einer weiteren bevorzugten Ausführungsform wird das erfindungsgemässe Fermentationsverfahren als Zulauf-Verfahren (auch bekannt als Fed-Batch-Prozess) durchgeführt; hierbei wird die Kohlenstoffquelle Ethanol fortlaufend limitiert zugeführt. Dabei kann die Konzentration des Ethanols im Fermentationsmedium insbesondere 0.0001 g/L - 2.5 g/L, bevorzugt 0.001 g/L - 2.5 g/L, besonders bevorzugt 0.01 g/L - 2.5 g/L betragen.

In Abhängigkeit von der gewählten Verfahrensführung kann die Konzentration des Ethanols im Fermentationsmedium somit zumindest zeitweise, insbesondere zu Beginn des Fermentationsverfahrens, im Bereich von 0.0001 g/L - 100 g/L liegen.

Über die Ethanolkonzentration kann die Gesamtmenge der Fettsäurenzusammensetzung gesteuert werden. Die Nutzung von Ethanol als Kohlenstoffquelle in der fermentativen Herstellung der Fettsäurenzusammensetzung hat zu einer überraschend hochwertigen Fettsäurenzusammensetzung geführt. Gleichzeitig war auch die Gesamtausbeute der Fettsäurenzusammensetzung hoch. Erste Resultate haben gezeigt, dass mittels adaptiver Laborevolution, also das wiederholte Kultivieren/Selektieren von Ölhefen unter hohen Ethanolkonzentrationen, Ölhefen mit Mutationen isolierbar sind, die Ethanol in Konzentrationen von bis zu 100 g/L nutzen können.

In einer bevorzugten Ausführungsform des erfindungsgemässen Fermentationsverfahrens umfasst das Fermentationsmedium eine Stickstoffquelle, wobei die Stickstoffquelle ausgewählt ist aus der Gruppe bestehend aus (NH₄)₂(SO₄), Alanin, Aspartat, Glutamat, Glutamin, Serin, Lysin.

(NH₄)₂(SO₄) hat den Vorteil, dass es keine zusätzliche Kohlenstoffquelle in das Fermentationsmedium einführt und der Stickstoff in einer gut bioverfügbaren Form vorliegt, wobei das (NH₄)₂(SO₄) sehr gut löslich ist.

Es hat sich gezeigt, dass die genannten Aminosäuren zu besonders guten Ausbeuten geführt haben. Ohne an diese Theorie gebunden zu sein, wird davon ausgegangen, dass dies daraus hervorgeht, dass die Aminosäuren mit zentralen Metaboliten der Ölhefe, insbesondere *Yarrowia Lipolytica,* über kurze Reaktionskaskaden verbunden sind. Dies kann zu einer Wachstumsverbesserung und/oder Ausbeuteverbesserung durch Steigerung der Verfügbarkeit der zentralen Metaboliten führen. Hierbei kann NAD(P)H freigesetzt werden, was von Vorteil für das Fermentationsverfahren ist. Es wird davon ausgegangen, dass die Vorteile der Nutzung der genannten Aminosäuren folgendermassen entstehen: Durch den Einsatz von Alanin, wird Pyruvat bereitgestellt, welches ein zentraler Metabolit beim Wachstum der Ölhefe, insbesondere *Yarrowia Lipolytica,* ist. Aspartat hat den Vorteil, dass bei dessen Abbau Oxalacetat bereitgestellt wird, was ein zentraler Metabolit des für das Wachstum der Hefe wichtigen Citratzyklus ist. Der Abbau von Glutamat führt zu der Produktion von NAD(P)H und weiteren wichtigen Metaboliten des Citratzyklus. Der Stickstoff wird hierbei als gut Bioverfügbares NH₃ bereitgestellt. Glutamin ist ein Präkursor von Nukleotiden, welche wichtig für das Wachstum der Ölhefe, insbesondere *Yarrowia Lipolytica,* sind. Das Serin hat den Vorteil, dass direkt Pyruvat und Ammoniak bereitgestellt werden kann. Durch Lysin kann NAD(P)H bereitgestellt werden, was, wie oben beschrieben, das Fermentationsverfahren und das Hefewachstum begünstigt.

In einer bevorzugten Ausführungsform des erfindungsgemässen Fermentationsverfahrens, beträgt das Molverhältnis von Kohlenstoff zu Stickstoff im Fermentationsmedium 5 - 100, bevorzugt 10 - 50, besonders bevorzugt 25 - 35.

Hierbei bedeutet das Molverhältnis das Verhältnis zwischen der molaren Konzentration an bioverfügbarem Kohlenstoffatomen und der molaren Konzentration an bioverfügbarem Stickstoffatomen gemeint. Dieses kann aus der folgenden Formel berechnet werden: $\frac{\frac{{conc}_{C}}{{M}_{C}} \times AC}{\frac{{conc}_{N}}{{M}_{N}} \times AN}$ wobei gilt:
conc_{C} = Konzentration der Kohlenstoffquelle in [g/L].
conc_{N} = Die Konzentration der Stickstoffquelle in [g/L].
M_{C} = Molare Masse der Kohlenstoffquelle in [g/mol].
M_{N} = Molare Masse der Stickstoffquelle in [g/mol].
AC = Die Anzahl Kohlenstoffatome in der Verbindung der Kohlenstoffquelle.
AN = Die Anzahl Stickstoffatome in der Verbindung der Stickstoffquelle.

Es wurde gefunden, dass das Molverhältnis von Kohlenstoff zu Stickstoff im Fermentationsmedium einen grossen Einfluss auf die Biomassenbildung und die Fettsäurenproduktion hat. Während ein hohes Verhältnis zu einer hohen Biomasseausbeute führt, kann ein tieferes Verhältnis zu einer erhöhten Fettsäurenausbeute führen. Das Verhältnis kann somit je nach Anwendung angepasst werden.

In einer bevorzugten Ausführungsform des erfindungsgemässen Fermentationsverfahrens stellt das Ethanol im Wesentlichen die einzige Kohlenstoffquelle im Fermentationsmedium dar.

Darunter wird verstanden, dass das Ethanol mindestens 85%, bevorzugt 90%, besonders bevorzugt 95% des für die Ölhefe, insbesondere für *Yarrowia Lipolytica,* bioverfügbaren Kohlenstoffs im Fermentationsmedium ausmacht.

In einer bevorzugten Ausführungsform des erfindungsgemässen Fermentationsverfahrens umfasst das Fermentationsmedium Hefeextrakt.

Die Zugabe von Hefeextrakt (CAS Nr. 8013012) in das Fermentationsmedium hat den Vorteil, dass die Wachstumsphase der Ölhefe deutlich verkürzt wird, wodurch der gesamte Prozess beschleunigt werden kann.

In einer bevorzugten Ausführungsform des erfindungsgemässen Fermentationsverfahrens ist die Ölhefe *Yarrowia Lipolytica.*

Die Nutzung von *Yarrowia Lipolytica,* insbesondere DSM 3286, als Ölhefe ist mit dem Vorteil verbunden, dass sie eine hochwertige Fettsäurenzusammensetzung herstellt, die in vielen Bereichen einsetzbar ist, beispielsweise für Nahrungsmittel, Kosmetik oder Treibstoff. Weiter vorteilhaft ist die Eigenschaft der Ölhefe *Yarrowia Lipolytica,* dass sie Ethanol besonders effektiv umsetzt. Hierbei sind Ethanolkonzentrationen von über 15 g/L im Fermentationsmedium möglich. Bei der Verwendung von mittels adaptiver Laborevolution erzeugter Mutanten kann die Ethanolkonzentration bis auf 100 g/L erhöht werden. Im Vergleich zu anderen Ölhefen, hat *Yarrowia Lipolytica* eine erhöhte Toleranz gegenüber externen physikalischen und chemischen Störfaktoren sowie mikrobiellen Verunreinigungen.

In einer bevorzugten Ausführungsform des erfindungsgemässen Fermentationsverfahrens umfasst das Fermentationsmedium Magnesiumionen, wobei die Konzentration der Magnesiumionen im Fermentationsmedium 0.8 mM - 16.5 mM beträgt, bevorzugt 5.0 mM - 16.0 mM, besonders bevorzugt 10.0 mM - 15.8 mM.

In einer bevorzugten Ausführungsform des erfindungsgemässen Fermentationsverfahrens wird der pH-Wert im Fermentationsmedium, insbesondere während der Wachstumsphase, im Wesentlichen konstant gehalten, insbesondere gepuffert.

Hierbei liegt der pH-Wert in einem Bereich von 4.0 - 7.0, bevorzugt 4.5 - 6.0, besonders bevorzugt 5.5 - 6.0. Es wurde festgestellt, dass ein im Wesentlichen konstanter pH-Wert von Vorteil für das Wachstum von Ölhefen, insbesondere *Yarrowia Lipolytica,* auf Ethanol mit Kaliumionen ist. Als "im Wesentlichen konstant", wird hierbei eine Schwankung von nicht mehr als ± 0.25 pH Einheiten verstanden.

Beispiele für geeignete Puffer sind: Essigsäure-Acetat-Puffer, Phosphatpuffer, Citratpuffer, PIPES (CAS Nr. 5625-37-6), HEPES (CAS Nr. 7356-45-9), MOPS (CAS Nr. 1132-61-2), Tris (CAS Nr. 77-86-1).

Die Pufferung ist insbesondere zu Beginn des Fermentationsverfahrens sinnvoll. Es ist möglich, dass während des Verfahrens Säuren entstehen, was die kontinuierliche Zugabe von Base bedingt, damit der pH-Wert im Fermentationsmedium konstant gehalten werden kann.

Es kann jedoch von Vorteil sein, den pH-Wert im Fermentationsmedium am Ende der Wachstumsphase auf einen Wert von 2.00 - 4.00, bevorzugt 2.50 - 3.50, besonders bevorzugt 2.75 - 3.25, zu senken. Das Fermentationsverfahren wird dann weitergeführt, wobei der pH in diesem gesenkten Wertebereich für 2.0 h - 6.0 h, bevorzugt 3.0 h - 4.0 h, besonders bevorzugt 3.5 h - 4.5 h, gehalten wird. Das Ende der Wachstumsphase ist dann erreicht, wenn die Ölhefe, insbesondere *Yarrowia Lipolytica,* mehr Energie für die Fettsäurenproduktion aufwendet als für die Zellteilung. Das Ende der Wachstumsphase kann beispielsweise zwischen 48 h und 72 h nach Beginn des Fermentationsverfahrens eintreten. Durch das Absenken des pH-Wertes am Ende der Wachstumsphase kann die Fettsäurenausbeute der Ölhefe, insbesondere *Yarrowia Lipolytica,* weiter erhöht werden.

Ein weiterer Aspekt der Erfindung betrifft die Verwendung von Kaliumionen in einem Fermentationsmedium zur Beeinflussung des Massenanteils von Fettsäuren in einer durch Fermentation mittels einer Ölhefe erhaltenen Fettsäurenzusammensetzung.

Dabei können die Kaliumionen in einem Fermentationsverfahren gemäss allen vorherigen Ausführungsformen verwendet werden. Die Ölhefe kann besonders bevorzugt *Yarrowia Lipolytica* sein.

Hierbei können die Massenanteile von Fettsäuren in der Fettsäurenzusammensetzung über die Konzentration der Kaliumionen beeinflusst werden. Im Konzentrationsbereich von 0.5 mM - 150 mM ist eine Erhöhung der Kaliumionenkonzentration allgemein mit einer höheren Fettsäureausbeute verbunden. Weiter wurde überraschend festgestellt, dass eine Erniedrigung der Kaliumionenkonzentration zu einem höheren Massenanteil der mehrfach ungesättigten Fettsäuren, insbesondere der Linolsäure, führt. Weiter kann eine Erniedrigung der Kaliumionenkonzentration zu einem niedrigeren Massenanteil an gesättigten Fettsäuren führen. Dadurch kann beispielsweise der Massenanteil der Linolsäure stark erhöht werden. Je nach Anwendung kann somit entweder die Gesamtmenge oder die Qualität der Fettäurenzusammensetzung über die Steuerung der Kaliumionenkonzentration erhöht werden.

In einer bevorzugten Ausführungsform der erfindungsgemässen Verwendung von Kaliumionen wird das Massenverhältnis von einfach ungesättigten zu mehrfach ungesättigten Fettsäuren [einfach ungesättigt]/[mehrfach ungesättigt], insbesondere von Ölsäure zu Linolsäure, beeinflusst.

Das Massenverhältnis ergibt sich experimentell aus der Fläche des mittels GC-MS bestimmten "Total Ion Chromatogramm"-Peaks (TIC-Peaks) der einfach ungesättigten Fettsäuren, insbesondere der Ölsäure, dividiert durch die Fläche des TIC-Peaks der mehrfach ungesättigten Fettsäuren, insbesondere der Linolsäure. Mehr Informationen dazu finden sich im ersten Ausführungsbeispiel. Die beschriebene Ausführungsform hat den Vorteil, dass bei der Verschiebung der Massenverhältnisse hin zu der Linolsäure eine Fettsäurenzusammensetzung erhalten wird mit einem höheren Massenanteil an mehrfach ungesättigten Fettsäuren. So kann man die Fettsäurenzusammensetzung je nach Anwendung und Qualitätsanforderungen anpassen.

In einer bevorzugten Ausführungsform der erfindungsgemässen Verwendung von Kaliumionen werden die Kaliumionen in einer derartigen Menge verwendet, dass die durch Fermentation erhaltene Fettsäurenzusammensetzung Ölsäure und Linolsäure in einem Massenverhältnis [Ölsäure]/[Linolsäure] im Bereich von 5 - 0.8, bevorzugt 3 - 1, besonders bevorzugt 2.5 - 1.3 enthält.

Ein weiterer Aspekt der Erfindung betrifft eine kosmetische Zusammensetzung, enthaltend eine Fettsäurenzusammensetzung erhalten durch ein Fermentationsverfahren gemäss einem der vorherigen Ansprüche.

Die Aufarbeitung der Fettsäurenzusammensetzung aus der durch das Fermentationsverfahren erhaltenen Biomasse kann im Labormassstab mit einer Chloroform/Methanol-Lösung (2:1) oder einer DMF/Methanol-Lösung (1:1) durchgeführt werden. Alternativ lässt sich die Extraktion auch mittels des Soxhlet-Verfahrens mit Hexan oder Cyclohexan durchführen. Bei der Extraktion von grösseren Mengen, kann die Methode der kontinuierlichen Extraktion, insbesondere der Gegenstromextraktion, angewandt werden.

Die kosmetische Zusammensetzung kann beispielsweise ausgewählt werden aus der folgenden Gruppe: Eine Balsamformulierung auf Ölbasis, eine flüssige Formulierung auf Ölbasis, eine wässrige kosmetische Formulierung, ein Duschgel, ein reinigendes Rückfettungspräparat auf Tensidbasis, ein Reinigungsöl für das Gesicht, ein Mizellenwasser, eine Cremedusche, ein Haaröl, ein ölbasierter Lippenpflegestift, ein After-Shower Körper Öl, eine Emulsion, ein Conditioner, ein Shampoo. Die erfindungsgemässe Fettsäurezusammensetzung zeichnet sich durch Ihre Eignung für den Einsatz in einer Vielzahl kosmetischer Anwendungen aus. Durch die Zugabe der erfindungsgemässen Fettsäurenzusamensetzung erhalten die Kosmetikprodukte eine verbesserte hautpflegende Wirkung.

Die Erfindung wird nun anhand verschiedener Ausführungsbeispiele beschrieben. Diese Ausführungsformen sollen den Kern der Erfindung in keiner Weise einschränken, sondern dienen vielmehr das Verständnis der Erfindung zu erleichtern.

### Ausführungsbeispiel 1

In Tabelle 1 sind die Inhaltsstoffe der Fermentationsmedien verschiedener Experimente aufgeführt, wobei das C/N Ratio über die Menge der Stickstoffquelle ((NH₄)₂(SO₄)) gesteuert wurde und die nicht genannten Inhaltsstoffe des Fermentationsmediums ansonsten konstant gehalten wurden. Weiter sind die zugehörigen End-Optischen-Dichten der Biomasse bei einer Wellenlänge von 600 nm gezeigt (End-OD₆₀₀, welche proportional zu der Biomasseausbeute sind) und die Bewertung des relativen Fettsäurengehalts im Fermentationsmedium zum Ende des Fermentationsverfahrens. Der relative Fettsäurengehalt beschreibt den Fettsäurengehalt eines Mediums (beurteilt anhand der Flächen des TICs im GC-MS) relativ zu den Fettsäurengehalten der anderen Medien. Gewertet wurde dies auf einer Skala von + bis ++++++, wobei + einen verhältnismässig tiefen Fettsäurengehalt und ++++++ einen verhältnismässig hohen Fettsäurengehalt bezeichnet. Dabei haben alle untersuchten Medien zu guten Fettsäurengehalten geführt und die Skala dient lediglich dem Vergleich der Medien untereinander. Es konnte gezeigt werden, dass mit Ethanol als Kohlenstoffquelle und Kaliumionen unerwarteterweise eine beachtliche Ausbeute an Fettsäuren erzielt werden kann.

**Tabelle 1:**

| **Medium** | **K⁺ [mM]** | **Mg²⁺ [g/L]** | **C/N** | **End-OD600** | **Rel. Fettsäurengehalt** |
|---|---|---|---|---|---|
| 1 | 75 | 1.05 | 15 | 10.8 | ++ |
| 2 | 87.55 | 1.9 | 15 | 11.7 | +++ |
| 3 | 87.55 | 1.05 | 35 | 8.4 | +++++ |
| 4 | 100 | 1.9 | 35 | 8.3 | +++++ |
| 5 | 75 | 1.9 | 25 | 11.3 | ++++++ |
| 6 | 87.55 | 1.9 | 35 | 8.3 | ++++ |
| 7 | 87.55 | 0.2 | 15 | 9.9 | +++++ |
| 8 | 100 | 1.05 | 15 | 10.8 | +++ |
| 9 | 100 | 1.9 | 25 | 10.8 | +++ |
| 10 | 100 | 0.2 | 25 | 9.5 | ++ |
| 11 | 87.55 | 1.05 | 25 | 10.5 | + |
| 12 | 87.55 | 0.2 | 35 | 7.9 | ++++ |
| 13 | 75 | 0.2 | 35 | 7.9 | ++++ |
| 14 | 75 | 0.2 | 25 | 8.7 | +++++ |
| 15 (Kontrolle) | 100 | 0.29 | 25.71 | 8.9 | +++ |
| 16 (Kontrolle) | 92.86 | 1.86 | 17.14 | 9.6 | +++++ |

Die Extraktion der Fettsäuren aus den gefriergetrockneten Hefepellets von *Yarrowia Lipolytica* wurde in Anlehnung an eine durch Folch et al. beschriebene Methode durchgeführt (J. Folch et al. A simple method for the isolation and purification of total lipides from animal tissues, J Biol Chem., Mai 1957; 226(1):497-509).

Die Extraktion wurde dabei folgendermassen durchgeführt:
1. Pro 10 mg gefriergetrocknetes Biomassepellet werden 1 ml Chloroform:Methanol (2:1 2(v/v)) zupipettiert (= 1. Normalisierung).
2. Mischen durch Vortexen (Stufe: max/ 10 sec/ Raumtemperatur (RT)).
3. Aufschliessen/extrahieren/homogenisieren mittels Ultraschallbad 30 min, 25 °C.
4. Mischen durch Vortexen (Stufe: max/ 10 sec/ RT).
5. Extraktion im Thermomix/Einsatz 2 ml Tubes (1000 rpm, 1 Stunde, 20 °C).
6. Zugabe von 500 µl 0,9 % (w/v) NaCl-Lösung.
7. Schonend mischen durch 10 x Schwenken/Invertieren der Tubes (RT).
8. Zentrifugation: 3800 g, 6 min, RT.
   Es entstehen 2 Phasen, sowie eine weisse Interphase. Obere Phase: wässrig. Interphase: Zelltrümmer. Untere Phase: organische Phase mit Fetten.
9. Abnahme der oberen (wässrigen) Phase mittels Pipette. Diese Phase wird verworfen.
10. Erneutes Zupipettieren von 500 µl 0,9 % (w/v) NaCl-Lösung zur unteren (unpolaren) Phase.
11. Schonend mischen durch 10 x Schwenken/Invertieren der Tubes (RT).
12. Zentrifugation: 3800 g, 6 min, RT. Es entstehen 2 Phasen, sowie eine weisse Interphase. Obere Phase: wässrig. Interphase: Zelltrümmer. Untere Phase: organische Phase mit Fetten.
13. Die Probennahme erfolgt aus der unpolaren Phase unterhalb der wässrigen Phase sowie unterhalb der weissen Interphase. Es werden pro Extraktion 50 µL der unteren, unpolaren Phase entnommen und in ein 1,5 ml Glas-GC-Vial überführt.
14. Trocknung der Proben im 1,5 ml Glas-GC-Vial (Trockenschrank, 60 °C, 15 min).
15. Rücklösen in Hexan: Zugabe von 500 µL n-Hexan, mischen durch vortexen (Stufe: max/ 30 sec/ RT).
16. Umesterung: Zugabe von 100 µl 2 N KOH in Methanol zupipettiert. Mischen durch vortexen (Stufe:max/60 sec/ RT).
17. Es entstehen 2 Phasen: Untere Phase (polare Phase: Wasser und Methanol). Obere Phase (unpolare Phase: n-Hexan mit Fettsäureestern). Überführen der oberen Phase in ein 1,5 ml Braunglas GC-Vial mit Glas-Inlet mittels Pipettieren (ca. 100 - 150 µl) und Verschliessen der Probe.
18. Die Proben werden anschliessend sofort an der GC-MS vermessen. Injektionsvolumen: 1 µl.

Aus diesem Extraktionsverfahren resultiert eine Fettsäurenzusammensetzung, wobei die Fettsäuren als Fettsäuremethylester (FAMEs) vorliegen. Die Analyse der Proben erfolgte mittels GC-MS. Hierbei wurden die FAMEs in einem ersten Schritt in einem GC-MS (GC-2010 Plus, Shimadzu; und GCMS-QP210 Plus, Shimadzu) aufgetrennt und analysiert, das mit einem Massenspektrometer (MS)-Detektor und einer HP-INNOWAX-Säule (Code: 19091N-136l) (60 m × 0,25 mm × 0,25 µm) ausgestattet war. Das Injektionsvolumen der Proben betrug 1 µL (splitless). Der Autosampler von Shimadzu hatte die Bezeichnung MB 01-00A REV7. Als Trägergas wurde Helium mit einer Flussrate von 1 mL/min verwendet. Die initiale Säulentemperatur lag bei 120 °C (für 3 min gehalten). Danach erfolgte die Auftrennung der Probenmatrix mit einem Temperaturgradienten: 10 °C/min auf 180 °C (für 19 min gehalten) und anschliessend mit 10 °C/min auf 250 °C erhöht (für 19 min gehalten). Als Referenz wurde die standarisierte NIST Datenbank (https://www.nist.gov/data) sowie der Supelco 37 Component FAME Mix (EG-Nummer: 200-838-9) als externer Standard genutzt.

Die Bestimmung der Massenanteile erfolgte mittels der Software GCMS Postrun Analysis (Version 4.52) von Shimadzu. Hierbei wurde eine Peak-Integration des "Total Ion Chromatogramms" (TICs) durchgeführt. Die Peaks der fünf häufigsten FAMEs wurden integriert, wobei isomere Formen desselben FAMEs zu einer Fläche zusammengerechnet wurden. Die erhaltenen Flächen der Peaks sind proportional zu der Masse der FAMEs. Zur Bestimmung des Massenanteils eines FAMEs wird die Fläche dieses FAMEs (FeF) durch die summierte Fläche aller gewerteten FAMEs (FaF) geteilt und in Prozent angegeben (FeS/FaF × 100). Dieser Wert entspricht dem Massenanteil eines bestimmten FAMEs. Es ist auch möglich, den Massenanteil einer Fettsäureart zu bestimmen, wie zum Beispiel der einfach ungesättigten Fettsäuren, indem die Fläche der TIC-Peaks aller Fettsäuren dieser Art summiert werden und durch die FaF geteilt werden. Falls notwendig, erfolgte eine manuelle Integration der Peaks zur Sicherstellung der Genauigkeit der Ergebnisse. Die identifizierten FAMEs wurden dann mit der NIST-Datenbank abgeglichen, um deren Identität zu bestätigen. Als Referenz diente der Supelco 37 Component FAME Mix (EG-Nummer: 200-838-9), welcher zur Validierung der Analyseergebnisse eingesetzt wurde. Die Bestimmung der Massenverhältnisse wurde analog vorgenommen, wobei man die Fläche einer Fettsäure oder Fettsäureart durch die Fläche einer anderen Fettsäure oder Fettsäureart geteilt hat. Der Fettsäurengehalt wurde analog bestimmt, wobei die FaFs der Medien miteinander verglichen wurden.

### Ausführungsbeispiel 2

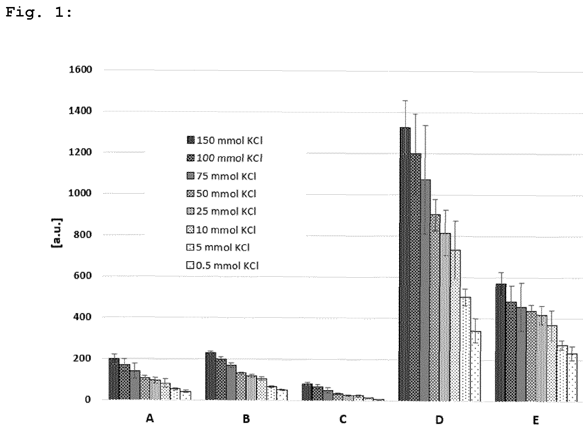

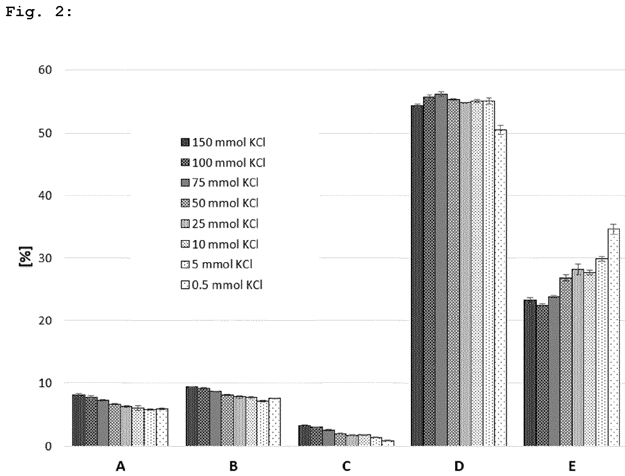

In Figuren 1 und 2 sind experimentelle Daten zum Einfluss der Kaliumionenkonzentration auf die Bildung der einzelnen Fettsäuren gezeigt. Hierbei entsprechen in Figur 1 und Figur 2 die Buchstaben den folgenden Fettsäuren:
- A: Palmitinsäure
- B: Palmitoleinsäure
- C: Stearinsäure
- D: Ölsäure
- E: Linolsäure.

Isomere Formen der Fettsäuren sind unter einem Buchstaben zusammengefasst. Untersucht wurden die Kaliumionenkonzentrationen im Bereich von 0,5 mM KCl bis 150 mM KCl, einschliesslich der Grenzwerte. Die absolute Fettsäurenausbeute in Abhängigkeit der Kaliumionenkonzentration ist in Figur 1 ersichtlich. Hierbei zeigt die vertikale y-Achse, mit [a.u.] beschriftet, die Flächen, welche aus der Peak-Integration des TICs resultierten. Dieser Wert ist proportional zu den Massen der gemessenen Fettsäuren und repräsentiert die absolute Fettsäurenausbeute. Eine höhere Kaliumionenkonzentration hat dabei über alle Fettsäuren hinweg zu einer höheren Fettsäurenausbeute geführt. Figur 2 zeigt die Massenanteile der einzelnen Fettsäuren in der Fettsäurenzusammensetzung bei unterschiedlichen Kaliumionenkonzentrationen. Die vertikale y-Achse, mit [%] beschriftet, bezeichnet den Massenanteil. Weiter konnte gezeigt werden, dass der Massenanteil der Fettsäuren überraschenderweise über die Kaliumionenkonzentration gesteuert werden kann. Insbesondere der Massenanteil der Linolsäure kann über eine Verringerung der Kaliumionenkonzentration erhöht werden. Gleichzeitig sinkt der Massenanteil der meisten anderen Fettsäuren bei tieferer Kaliumionenkonzentration. Somit konnte gezeigt werden, dass bei der fermentativen Herstellung von Fettsäuren, bei der Nutzung von Ethanol als Kohlenstoffquelle, der Massenanteil der mehrfach ungesättigten Fettsäuren, insbesondere Linolsäure, über die Kaliumionenkonzentration gesteuert werden kann.

### Ausführungsbeispiel 3

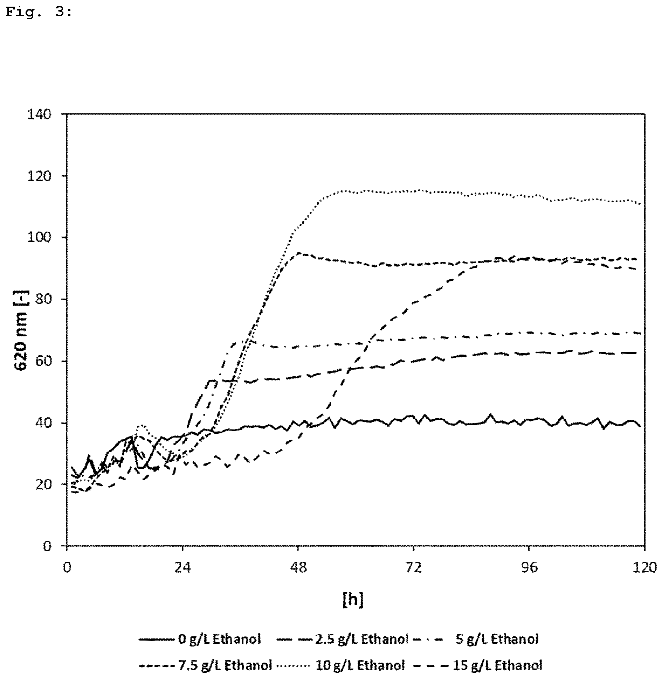

In Figur 3 werden experimentelle Daten zu dem Einfluss der Ethanolkonzentration auf das Wachstum der Hefe gezeigt. Die Menge an Biomasse wurde mittels Rückstreuung bei 620 nm quantifiziert. Die Rückstreuung ist proportional zur Menge an Biomasse und eignet sich daher zum Vergleich verschiedener Wachstumsprofile der Hefe. Die Rückstreuung ist anhand einer relativen Einheit an der vertikalen y-Achse abzulesen. Auf der horizontalen x-Achse ist die Zeitdauer der Hefenkultivierung in Stunden ersichtlich.

Es wurden Ethanolkonzentrationen von 0 - 15 g/L untersucht. Bei 10 g/L war das Wachstum besser als bei 7.5 g/L und besser als bei 15 g/L. In einer Konzentration von 15 g/L scheint das Ethanol eine hemmende Wirkung auf das Wachstum der Hefe zu haben. Bei 10 g/L Ethanol wurde zudem eine kürzere Wachstumsphase beobachtet als bei 15 g/L.

### Ausführungsbeispiel 4

In Ausführungsbeispiel 4 wurde die Eignung der Fettsäurenzusammensetzung als Inhaltsstoff in verschiedenen kosmetischen Produkten getestet. Zu diesem Zweck wurde eine Nachbildungszusammensetzung hergestellt, welche Ölsäure (58 Gew. %), Linolsäure (23 Gew. %), Palmitinsäure (14 Gew. %) und Stearinsäure (5 Gew. %) enthält. Die Gewichtsverhältnisse der vier Fettsäuren zueinander wurden im Vergleich zu der originalen Fettsäurenzusammensetzung beibehalten. Die Nachbildungszusammensetzung wurde in den folgenden kosmetischen Produkten eingesetzt:
- Balsamformulierung auf Ölbasis
- flüssige Formulierung auf Ölbasis
- wässrige kosmetische Formulierung
- Duschgel
- reinigendes Rückfettungspräparat auf Tensidbasis

Die ersten drei kosmetischen Produkte wurden ausgehend von einer Referenz hergestellt, welche typische Inhaltsstoffe für die jeweiligen Produkte umfasst. Dabei wurden einzelne Inhaltsstoffe der Referenzen durch die Nachbildungszusammensetzung ersetzt. Die exakte Zusammensetzung der kosmetischen Produkte ist in Tabelle 2 ersichtlich. In Tabelle 2 und Tabelle 3 wurden, wenn nicht anders erwähnt, die INCI-Bezeichnungen der Inhaltsstoffe eingetragen.

**Tabelle 2:**

| *Balsamformulierung auf Ölbasis* | | |
|---|---|---|
| **Inhaltsstoff** | **Mengen kosmetisches Produkt (Gew. %)** | **Mengen Referenz (Gew. %)** |
| Helianthus Annuus Hybrid Oill | 74.6 | 74.6 |
| Hydrogenated Castor Oil | 6 | 6 |
| **Octyldodecanol** | - | 5 |
| **Butyrospermum Parkii Butter** | - | 3 |
| **Nachbildungszusammensetzung** | **8** | - |
| Cera Alba | 2.5 | 2.5 |
| Hydrogenated Palm Oil | 2.5 | 2.5 |
| Hydrogenated Rapeseed Oil | 2.5 | 2.5 |
| Polyglyceryl-2 Di-Polyhydroxystearate | 1.5 | 1.5 |
| Tocopheryl Acetate | 1 | 1 |
| Vitis Vinifera Seed Oil | 1 | 1 |
| Aroma | 0.4 | 0.4 |
| *Flüssige Formulierung auf Ölbasis* | | |

| **Inhaltsstoff** | **Mengen kosmetisches Produkt (Gew.** %) | **Mengen Referenz (Gew.** %) |
|---|---|---|
| Helianthus Annuus Hybrid Oil | 35.4 | 35.4 |
| Oryza Sativa Bran Oil | 25 | 25 |
| Olea Europaea Fruit Oil | 10 | 10 |
| Dicaprylyl Ether | 9.997 | 9.997 |
| **Sesamum Indicum Seed Oil** | - | **8** |
| **Nachbildungszusammensetzung** | **8** | - |
| Prunus Amygdalus Dulcis Oil | 6 | 6 |
| Macadamia Integrifolia/Tetra phylla Seed Oil | 4 | 4 |
| Parfum | 0.6 | 0.6 |
| Tocopheryl Acetate | 0.5 | 0.5 |
| Tocopherol | 0.2046 | 0.2046 |
| Helianthus Annuus Seed Oil | 0.1994 | 0.1994 |
| Olus Oil | 0.0984 | 0.0984 |
| Cl 40800 | 0.0006 | 0.0006 |

| *Wässrige kosmetische Formulierung* | | |
|---|---|---|
| **Inhaltsstoff** | **Mengen kosmetisches Produkt (Gew. %)** | **Mengen Referenz (Gew. %)** |
| Aqua | 98.3 | 98.3 |
| PEG-7 GlycerilCocoate | 1 | 1 |
| Sodium Benzoate | 0.5 | 0.5 |
| Citric Acid | 0.1 | 0.1 |
| **Macadamia Integrifolia/Tetra phylla Seed Oil** | - | **0.1** |
| **Nachbildungszusammensetzung** | **0.1** | - |

| *Duschgel* | | |
|---|---|---|
| **Inhaltsstoff** | **Mengen kosmetisches Produkt (Gew. %)** | |
| Aqua | 74.0075 | |
| Sodium Laureth Sulfate | 9.03 | |
| Glycerin | 7.1625 | |
| **Nachbildungszusammensetzung** | **3** | |
| Cocamidopropyl Betaine | 2.3075 | |
| Sodium Chloride | 1.9225 | |
| PEG-7 Glyceryl Cocoate | 1 | |
| Parfum | 0.7 | |
| Sodium Benzoate | 0.4 | |
| Guar Hydroxypropyltrimonium Chloride | 0.27 | |
| Citric Acid | 0.2 | |

| *Reinigendes Rückfettungspräparat auf Tensidbasis* | | |
|---|---|---|
| **Inhaltsstoff** | **Mengen kosmetisches Produkt (Gew. %)** | |
| Aqua | 32.77275 | |
| **Nachbildungszusammensetzung** | **23** | |
| Cocamidopropyl Betaine | 20.96275 | |
| PEG-7 Glyceryl Cocoate | 17 | |
| Sodium Chloride | 3.83825 | |
| Glycerin | 1.47625 | |
| Citric Acid | 0.6 | |
| Sodium Benzoate | 0.35 | |

Bei der Balsamformulierung auf Ölbasis wurde im Vergleich zu der Referenz Octyldodecanol und Butyrospermum Parkii Butter durch gleiche Gewichtsprozente der Nachbildungszusammensetzung ausgetauscht. Die Viskosität des Balsams wurde durch die Modifikation nicht beeinflusst. Das Gefühl beim Auftragen auf die Haut veränderte sich von einer schmelzenden Textur zu einer samtigeren Textur. Es wurden keine Anzeichen von Instabilität nach 7 Monaten bei Raumtemperatur beobachtet.

Bei der flüssigen Formulierung auf Ölbasis, wurde im Vergleich zu der Referenz ein Pflanzenöl mit gleichen Gewichtsprozenten der Nachbildungszusammensetzung ausgetauscht. Die Fettsäuren lösten sich vollständig in der Formulierung, und das Ergebnis war ein klares Gesichtsöl ohne optische Abweichungen im Vergleich zur Referenzformulierung. Es wurden keine Anzeichen von Instabilität nach 7 Monaten bei Raumtemperatur beobachtet.

Bei der wässrigen kosmetischen Formulierung wurde im Vergleich zu der Referenz ein Pflanzenöl mit gleichen Gewichtsprozenten der Nachbildungszusammensetzung ersetzt.

Für das Duschgel wurde eine Duschgelformulierung verwendet, die Wasser, verschiedene Tenside, einen Lösungsvermittler, Glycerin, einen quaternären Konditionierungszusatz, ein Konservierungsmittel und ein Parfüm beinhaltet. Der Versuch zeigte, dass die Nachbildungszusammensetzung mit dem Lösungsvermittler PEG-7 Glycerin-Kokosat vorgemischt und den Tensiden zugegeben werden kann, was zu einem opaleszierenden Effekt führt.

Bei dem reinigenden Rückfettungspräparat auf Tensidbasis wurde ein Gel auf Wasserbasis erhalten, mit einem hohen Anteil der Nachbildungszusammensetzung (11,5 Gew. %). Das Gel blieb über einen Zeitraum von 7 Monaten bei Raumtemperatur stabil. Das Gel zeigte eine spürbare hautpflegende Wirkung, wenn es nach dem Händewaschen aufgetragen und abgespült wurde.

### Ausführungsbeispiel 5

In Ausführungsbeispiel 5 wurde eine sensorische Analyse anhand einer Öl/Wasser Emulsion enthaltend 5 Gew. % einer Fettsäurenzusammensetzung durchgeführt. Dabei wurden 5 Varianten der Emulsion geprüft. Die Varianten wurden gegenüber einer Referenzzusammensetzung getestet, welche Wasser statt der Fettsäurenzusammensetzung enthielt. Die geprüften Varianten enthielten: A. die erfindungsgemässe Fettsäurenzusammensetzung, erhalten durch fermentative Herstellung von Fettsäuren durch *Yarrowia Lipolytica,* B. eine Kombination aus Palmitinsäure und Stearinsäure in gleichen Gew. %, C. nur Ölsäure, D. nur Linolsäure oder E. die Nachbildungszusammensetzung. Die exakte Zusammensetzung ist in Tabelle 3 ersichtlich. In Tabelle 3 wurden für die Varianten A - E keine INCI-Namen verwendet.

**Tabelle 3:**

| **Inhaltsstoff** | **Anteil (Gew. %)** |
|---|---|
| Aqua | 74.58 |
| Helianthus Annuus Hybrid Oil | 8 |
| Glycerin | 3 |
| Referenz: Aqua | (5) |
| Variante A: erfindungsgemässe Fettsäurenzusammensetzung | (5) |
| Variante B: Stearinsäure/Palmitinsäure 1 zu 1 | (5) |
| Variante C: Ölsäure | (5) |
| Variante D: Linolsäure | (5) |
| Variante E: Nachbildungszusammensetzung | (5) |
| Butyrospermum Parkii Butter | 2 |
| Behenyl Alcohol | 1.5 |
| Cetyl Alcohol | 1.5 |
| Glyceryl Searate Citrate | 1.5 |
| Decyl Oleate | 1.499625 |
| Caprylyl Glycol | 0.8 |
| Xanthan Gum | 0.3 |
| Citric Acid | 0.2 |
| Sodium Hydroxide | 0.09 |
| Tocopherol | 0.020385 |
| Olus Oil | 0.00984 |
| Hydrogenated Palm Glycerides Citrate | 0.00015 |

Die Bewertung der Parameter Verteilbarkeit, Geschmeidigkeit, Reichhaltigkeit und Leuchtkraft erfolgte durch ein Panel von fünf geschulten Experten. Die Varianten A bis E wurden randomisiert mit den Nummern 1 bis 5 codiert, um eine verblindete Bewertung zu gewährleisten. Die Referenzemulsion (Variante R) wurde vorab appliziert und diente mit dem normierten Wert 0,0 als Referenz für alle vier untersuchten Eigenschaften. Die Testvarianten A bis E wurden anschliessend relativ zu der Referenz auf einer fünfstufigen Skala von -2 bis +2 bewertet. Aus den Einzelbewertungen der Probanden wurde der Mittelwert gebildet. Die Ergebnisse sind in Tabelle 4 ersichtlich.

**Tabelle 4:**

| **Attribut** | **Variante A** | **Variante B** | **Variante C** | **Variante D** | **Variante E** |
|---|---|---|---|---|---|
| verteilbar | 0 | -1.8 | 1 | -0.4 | -2 |
| geschmeidig | -0.6 | -1.6 | -1 | 0.4 | -1.6 |
| reichhaltig | 0.8 | 1.4 | 0.8 | 1 | 1.6 |
| leuchtkräftig | 1 | -0.8 | 0 | 0.8 | 0.6 |

Die erfindungsgemässe Fettsäurenzusammenestzung zeigte kosmetisch günstige Effekte in einer Konzentration von 5 Gew. % in Variante A. Variante A erhielt die Verteilbarkeit der Emulsion und trug gleichzeitig zu einem reichhaltigeren Hautgefühl sowie einer verbesserten Hautleuchtkraft bei, ohne die Geschmeidigkeit stark zu beeinträchtigen.

Variante B, formuliert mit Palmitin- und Stearinsäure, zeigte eine reduzierte Verteilbarkeit, was zu einer wachsartigen Textur mit eingeschränkter Geschmeidigkeit führte. Die Applikation resultierte in einer matten Hauterscheinung mit einem Fettfilm und einer erhöhten Reichhaltigkeit.

Die Variante C, formuliert mit Ölsäure, zeigte die höchste Verteilbarkeit im Vergleich aller getesteten Muster. Diese verbesserte Verteilbarkeit ging jedoch mit einem deutlichen Verlust an Geschmeidigkeit einher.

Die linolsäurehaltige Variante D war die einzige Formulierung, die gleichzeitig positive Effekte auf die Geschmeidigkeit, die Reichhaltigkeit und die Leuchtkraft zeigte. Diese sensorischen Verbesserungen gingen jedoch mit einer reduzierten Verteilbarkeit einher.

Die Variante E, formuliert mit der Nachbildungszusammensetzung, zeigte, dass der nicht näher spezifizierte Anteil der erfindungsgemässen Fettsäurenzusammensetzung einen signifikanten Einfluss auf die sensorische Gesamtwahrnehmung hat. Im Vergleich zu der Nachbildungszusammensetzung führte die erfindungsgemässe Zusammensetzung zu einer deutlich verbesserten Verteilbarkeit und Geschmeidigkeit. Diese Vorteile ergeben sich aus dem nicht nachgebildeten Gewichtsanteil der erfindungsgemässen Zusammensetzung, welcher die beiden Zusammensetzungen unterschiedet. Dieser unspezifizierte Anteil besteht hauptsächlich aus weiteren Fettsäuren und Lipiden.

## Patentansprüche

1. Fermentationsverfahren zur Erzeugung einer Fettsäurenzusammensetzung, umfassend die Schritte:
a. Bereitstellen eines Fermentationsmediums, das Ethanol und Kaliumionen enthält; und
b. Durchführung einer Fermentation mittels einer Ölhefe in dem Fermentationsmedium.

2. Fermentationsverfahren nach Anspruch 1, wobei die Konzentration der Kaliumionen im Fermentationsmedium 0.5 mM - 150 mM beträgt, bevorzugt 30 mM - 110 mM, besonders bevorzugt 50 mM - 80 mM.

3. Fermentationsverfahren nach einem der Ansprüche 1 oder 2, wobei der Schritt a. das Auflösen eines Kaliumsalzes umfasst, und wobei das Kaliumsalz ausgewählt ist aus der Gruppe bestehend aus KCl, K₂CO₃, KOH, K₂SO₄, K₃PO₄, CH₃COOK, C₆H₅K₃O₇ und HCOOK.

4. Fermentationsverfahren nach einem der vorstehenden Ansprüche, das
- als Chargenprozess durchgeführt wird, wobei die Ausgangskonzentration des Ethanols im Fermentationsmedium insbesondere 2.5 g/L - 100.0 g/L oder 2.5 g/L - 50.0 g/L oder 2.5 g/L - 15.0 g/L, bevorzugt 5.0 g/L - 13.0 g/L, besonders bevorzugt 8.0 g/L - 11.0 g/L beträgt; oder
- als Zulaufverfahren durchgeführt wird, wobei die Konzentration des Ethanols im Fermentationsmedium insbesondere im Bereich von 0.0001 g/L - 2.5 g/L, bevorzugt 0.001 g/L - 2.5 g/L, besonders bevorzugt 0.01 g/L - 2.5 g/L liegt.

5. Fermentationsverfahren nach einem der vorstehenden Ansprüche, wobei das Fermentationsmedium eine Stickstoffquelle umfasst, wobei die Stickstoffquelle ausgewählt ist aus der Gruppe bestehend aus (NH₄)₂(SO₄), Alanin, Aspartat, Glutamat, Glutamin, Serin, Lysin.

6. Fermentationsverfahren nach einem der vorstehenden Ansprüche, wobei das Molverhältnis von Kohlenstoff zu Stickstoff im Fermentationsmedium 5 - 100 beträgt, bevorzugt 10 - 50, besonders bevorzugt 25 - 35.

7. Fermentationsverfahren nach einem der vorstehenden Ansprüche, wobei das Ethanol im Wesentlichen die einzige Kohlenstoffquelle im Fermentationsmedium darstellt.

8. Fermentationsverfahren nach einem der vorstehenden Ansprüche, wobei das Fermentationsmedium Hefeextrakt umfasst.

9. Fermentationsverfahren nach einem der vorstehenden Ansprüche, wobei die Ölhefe *Yarrowia Lipolytica* ist.

10. Fermentationsverfahren nach einem der vorstehenden Ansprüche, wobei das Fermentationsmedium Magnesiumionen umfasst, und wobei die Konzentration der Magnesiumionen im Fermentationsmedium 0.8 mM - 16.5 mM beträgt, bevorzugt 5.0 mM - 16.0 mM, besonders bevorzugt 10.0 mM - 15.8 mM.

11. Fermentationsverfahren nach einem der vorstehenden Ansprüche, wobei der pH-Wert im Fermentationsmedium, insbesondere während der Wachstumsphase, im Wesentlichen konstant gehalten, insbesondere gepuffert wird.

12. Verwendung von Kaliumionen in einem Fermentationsmedium zur Beeinflussung der Massenanteile von Fettsäuren in einer durch Fermentation mittels einer Ölhefe, insbesondere *Yarrowia Lipolytica,* erhaltenen Fettsäurezusammensetzung.

13. Verwendung von Kaliumionen nach Anspruch 12, wobei das Massenverhältnis von einfach ungesättigten zu mehrfach ungesättigten Fettsäuren, insbesondere von Ölsäure zu Linolsäure, beeinflusst wird.

14. Verwendung von Kaliumionen nach Anspruch 12 in einer derartigen Menge, dass die durch Fermentation erhaltene Fettsäurenzusammensetzung Ölsäure und Linolsäure in einem Massenverhältnis Ölsäure zu Linolsäure im Bereich von 5 - 0.8, bevorzugt von 3 - 1, besonders bevorzugt von 2.4 - 1.3 enthält.

15. Kosmetische Zusammensetzung, enthaltend eine Fettsäurenzusammensetzung erhalten durch ein Fermentationsverfahren gemäss einem der vorherigen Ansprüche.
